(19) Europäisches Patentamt / European Patent Office / Office européen des brevets

(11) **EP 2 034 308 B1**

(12) **FASCICULE DE BREVET EUROPEEN**

(45) Date de publication et mention
de la délivrance du brevet:
**21.03.2012 Bulletin 2012/12**

(21) Numéro de dépôt: **08290731.2**

(22) Date de dépôt: **28.07.2008**

(51) Int Cl.:
*G01N 33/24* (2006.01)     *G01N 15/08* (2006.01)
*G01N 27/04* (2006.01)     *G01V 3/32* (2006.01)

(54) **Méthode de mesure rapide de la saturation et de la résistivité d'un milieu poreux**

Schnellmessverfahren der Sättigung und des Volumenwiderstands eines porösen Milieus

Method for rapidly measuring the saturation and resistivity of a porous medium

(84) Etats contractants désignés:
**GB NL NO**

(30) Priorité: **07.09.2007 FR 0706288**

(43) Date de publication de la demande:
**11.03.2009 Bulletin 2009/11**

(73) Titulaire: **IFP Energies nouvelles
92852 Rueil-Malmaison Cedex (FR)**

(72) Inventeurs:
• **Fleury, Marc
78170 La Celle-Saint-Cloud (FR)**
• **Han, Mei
75019 Paris (FR)**

(56) Documents cités:
EP-A- 0 586 001     EP-A- 1 398 630
EP-A- 1 729 151     FR-A- 2 844 355
FR-A- 2 864 244

• FLEURY M. ET AL: "A New Approach to Derive Relative Permeability Data while Measuring Resistivity Index" INTERNATIONAL SYMPOSIUM OF THE SOCIETY OF CORE ANALYSTS, août 2005 (2005-08), XP002474993 Toronto

## Description

La présente invention concerne le domaine pétrophysique et plus précisément la détermination de la saturation en saumure au sein d'un milieu poreux, tel qu'une formation géologique par exemple.

La connaissance de la saturation en saumure permet de calculer les quantités d'hydrocarbures en place dans le cas d'un réservoir pétrolier, ou la quantité de $CO2$ stockée dans le cas d'un stockage de $CO2$. Il est donc très important pour le spécialiste en charge de l'exploitation d'une formation souterraine, dans le cadre de production d'hydrocarbure ou de stockage de gaz acides par exemple, de connaître la saturation en saumure de cette formation.

## État de la technique

Il est connu de déterminer la saturation d'une formation géologique en réalisant des mesures électriques au sein de puits, en cours ou non de forage. Ces mesures au sein d'un puit sont appelées des diagraphies (« well logs »). Ces diagraphies électriques mesurent la résistivité électrique du milieu. Elles doivent être analysées de façon à déduire les valeurs de saturation au sein de la formation.

Une méthode classique pour réaliser une telle interprétation des diagraphies de résistivité consiste à utiliser les lois d'Archie, et notamment la relation suivante :

$$RI = \frac{R_t}{R_0} = S_w^{-n}$$

avec :

$RI$ : indice de résistivité

$S_w$ : saturation en saumure du milieu

$R_t$ : résistivité électrique du milieu

$R_0$ : résistivité électrique du milieu saturé à 100% par la saumure ($S_w = 1$).

$n$ : un nombre entier

La résistivité électrique du milieu $R_t$ est mesurée par diagraphies électriques, tandis que la résistivité électrique du milieu saturé à 100% par la saumure $R_0$, est déterminée à partir de mesures effectuées sur des carottes.

Une étape importante de l'interprétation des diagraphies de résistivité ($R_t$) en terme de saturation en saumure ($S_w$), consiste donc à définir le paramètre $n$.

Pour ce faire, il est connu de mesurer d'une part l'indice de résistivité et la saturation en saumure sur des échantillons issus de la formation géologique. Ces mesures sont souvent réalisées dans les conditions de pression et de température rencontrées au sein de la formation. Ainsi, en mesurant plusieurs couples ($RI, S_w$), on peut estimer le paramètre $n$ de la relation d'Archie.

La mesure de l'indice de résistivité d'un milieu poreux constitue l'étape expérimentale la plus difficile dans ce processus d'interprétation de la réponse électrique des milieux poreux.

Une technique disponible pour mesurer l'indice de résistivité et la saturation en saumure sur un grand nombre d'échantillons, consiste à placer ces échantillons sur une plaque poreuse semi-perméable à l'eau, l'ensemble étant disposé dans une enceinte pouvant être amenée en pression. Lorsque la pression P dans l'enceinte est augmentée progressivement, la saturation des échantillons diminue progressivement. A chaque palier de pression, on retire les échantillons de l'enceinte et on détermine la saturation par pesée. La résistivité est mesurée en appliquant deux plaques métalliques connectées à un appareil de mesure aux deux faces de l'échantillon.

Cependant, il est connu que la détermination de la saturation par cette technique est entachée d'erreurs dues à la perte de grain lors de la manipulation, et que la mesure de résistivité est entachée d'erreurs dues aux résistances de contact entre les plaques et le milieu poreux.

De plus, pour les échantillons peu perméables, une plaque poreuse de très faible perméabilité est nécessaire, nécessitant des durées d'expériences longues (de l'ordre de 2 mois au moins). L'utilisation d'une telle plaque poreuse pour mesurer des caractéristiques physiques d'échantillon solide poreux (S) est décrite dans le document FR-A-2 864 244.

**La méthode selon l'invention**

Ainsi, l'objet de l'invention concerne une méthode pour construire une relation permettant de déduire une saturation en un fluide conducteur en fonction d'une résistivité, pour un milieu poreux initialement saturé par ledit fluide, selon la revendication 1.

D'autres caractéristiques et avantages de la méthode selon l'invention, apparaîtront à la lecture de la description ci-après d'exemples non limitatifs de réalisations, en se référant aux figures annexées et décrites ci-après.

**Présentation succincte des figures**

- la figure 1 schématise les étapes de la méthode permettant de mesurer rapidement la saturation en un fluide conducteur et l'indice de résistivité d'un milieu poreux.

- la figure 2 schématise la mise en oeuvre de la désaturation par centrifugation, selon l'invention.

- la figure 3 illustre schématiquement un profil de saturation $S_w$ dans les échantillons $E1$ et $E2$ en contact capillaire, dans le cadre d'une centrifugation dans un dispositif selon la figure 2.

- la figure 4 montre des profils de saturation $S_w$ dans les échantillons $E1$ et $E2$, pour différentes vitesses de rotation de centrifugation.

- les figures 5A et 5B montrent un dispositif de mesure radiale de la résistivité.

- la figure 6 montre un exemple de résultat de mesure sur deux Grès de Fontainebleau de grande perméabilité.

- la figure 7 monte des mesures d'indice de résistivité et de saturation sur un calcaire à structure bimodale en drainage.

- la figure 8 monte des mesures d'indice de résistivité et de saturation sur un calcaire à structure bimodale en drainage.

**Description détaillée de la méthode**

La méthode selon l'invention permet de mesurer rapidement la saturation en un fluide conducteur et l'indice de résistivité d'un milieu poreux.

Habituellement, ces mesures sont réalisées dans le but d'obtenir des mesures extrêmement fiables, peu importe le temps nécessaire pour effectuer ces mesures. La méthode selon l'invention, au contraire, propose une méthode permettant d'obtenir rapidement ces mesures sans perte de précision.

La méthode de mesure est décrite dans le cadre d'un milieu contenant les deux fluides suivant : saumure et air. Cependant, la méthode est également applicable au milieu contenant de la saumure et de l'huile, comme un réservoir pétrolier par exemple, et même à tout milieu contenant au moins un fluide conducteur.

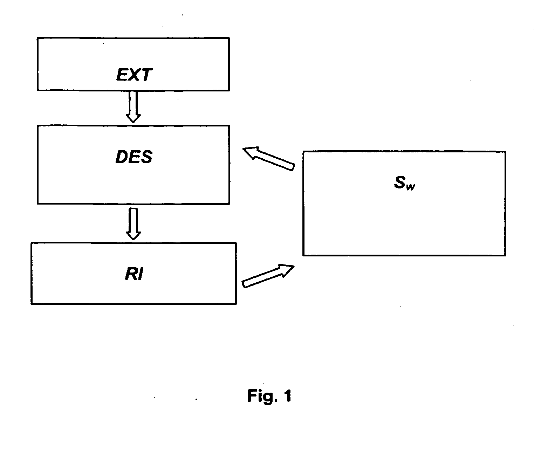

Pour un milieu contenant de la saumure (fluide conducteur) et de l'air, et initialement saturé par la saumure, on applique la procédure suivante, décrite sur la figure 1 :

- on extrait au moins un échantillon solide du milieu (en général une carotte) - *EXT*

- on désature partiellement l'échantillon par centrifugation - *DES*

- on mesure l'indice de résistivité par mesure radiale - *RI*

- on mesure la saturation à l'aide d'un appareil RMN - $S_w$

1- Désaturation partielle de l'échantillon

Le but de cette étape est d'obtenir une saturation moyenne $S_w$ dans l'échantillon. La technique utilisée consiste à drainer l'échantillon par centrifugation, selon un temps fixé, et selon une vitesse de rotation $\omega$ fixée, de façon à désaturer partiellement l'échantillon, jusqu'à obtenir une valeur particulière, mais inconnue, de saturation $S_w$.

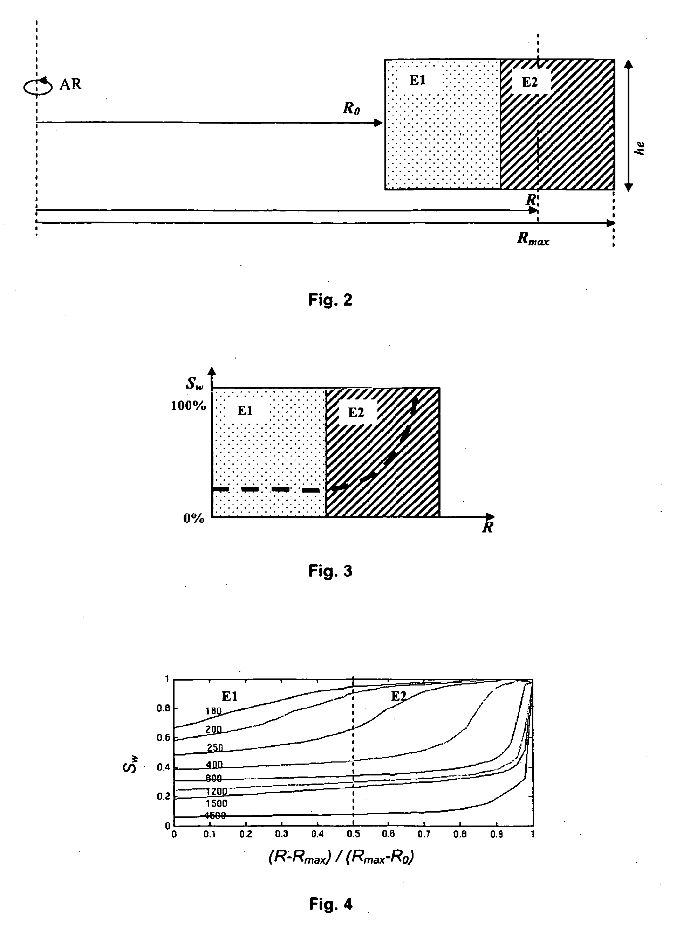

La figure 2 schématise la mise en oeuvre de la désaturation par centrifugation, selon l'invention. L'axe de rotation de la centrifugeuse (*AR*) est représenté à gauche. A titre d'exemple, les longueurs des échantillons sont identiques (0.03m), et leurs hauteurs (diamètres s'il s'agit de carottes cylindriques) également (*he*=0,04m). La distance *R* représente une distance par rapport à l'axe de rotation (*AR*). Deux valeurs particulières sont indiquées sur la figure 2 : la distance $R_0$ indique la distance entre cette axe et l'échantillon placé au sein de la cellule de centrifugation, au plus proche de l'axe *AR*, c'est-à-dire l'échantillon 1 (*E1*) ; la distance $R_{max}$ représente la distance entre l'axe de rotation (*AR*) et l'extrémité la plus éloignée de l'échantillon 2 (*E2*). Selon un exemple, $R_{max}$ = 0,263m.

Lorsqu'un échantillon saturé d'un fluide 1 est centrifugé dans un autre fluide 2 de densité différente, une différence de pression $\Delta P$ apparaît, produisant un changement de saturation de l'échantillon. La différence de pression générée est une fonction de la distance *R* par rapport au centre de rotation, de la vitesse de rotation ω et des densités des fluides. Cette pression est donnée par

$$\Delta P (R) = \frac{1}{2} \omega^2 \left( R_{\max}^2 - R^2 \right)\left( \rho_w - \rho_g \right) \qquad (1)$$

où $\rho_w$ et $\rho_g$ sont les densités des deux fluides (saumure, air dans ces expériences) et $R_{max}$. Lorsque la pression générée (Eq 1) liée à la rotation est supérieure à la pression capillaire, la saumure s'écoule. La saturation en saumure diminue jusqu'à un équilibre, obtenu quand le débit est nul. A l'équilibre et pour une pression donnée, la saturation est donnée par la courbe de pression capillaire.

En effectuant la centrifugation à différentes vitesses de rotation, on obtient ainsi différentes saturations. Il n'est cependant pas nécessaire d'atteindre l'équilibre capillaire.

D'après l'équation (1), la pression maximale (la saturation la plus faible) est obtenue à la face d'entrée ($R_0$ en drainage) du fluide de plus faible densité. Sur la face de sortie, la pression capillaire est nulle et ainsi, la saturation est théoriquement égale à un. La zone à saturation élevée constitue le pied capillaire. A cause de cette inhomogénéité de pression, le profil de saturation n'est pas uniforme le long de l'axe de rotation, comme le montre schématiquement la figure 3, qui illustre un profil de saturation $S_w$ dans les échantillons *E1* et *E2* en contact capillaire, en fonction du rayon *R*

Pour éviter des problèmes d'interprétation des mesures, liés à la saturation non uniforme dans l'échantillon, on superpose par exemple deux échantillons voisins issus d'une même carotte. Puis, on mesure les valeurs moyennes de saturation et de résistivité de l'échantillon 1.

Pour que l'échantillon 1 ait un profil de saturation uniforme, il est nécessaire d'avoir un bon contact capillaire avec l'échantillon 2. A cette fin, on peut utiliser une membrane mouillable à la saumure entre les deux échantillons. A cette fin également, il est préférable que l'échantillon 1 n'ait pas un diamètre supérieur à l'échantillon 2 (*he*, figure 2). On obtient typiquement des profils de saturation comme le montre la figure 4. Cette figure montre des profils de saturation $S_w$ dans les échantillons *E1* et *E2*, en fonction d'une longueur normalisée (($R$-$R_{max}$) / ($R_{max}$-$R_0$)), pour différentes vitesses de rotation de centrifugation (de 180 tour/min (courbe du haut) à 4500 tour/min (courbe du bas)).

On peut toutefois remplacer l'échantillon 2 par un autre échantillon issu d'un autre milieu, pourvu que sa porosité et sa perméabilité soient sensiblement identiques à celles de l'échantillon 1.

Ainsi, selon l'invention, on désature l'échantillon 1 dans une cellule de centrifugation, à côté d'un échantillon 2 adapté à limiter le pied capillaire dans l'échantillon 1. Pour ce faire, les échantillons sont disposés l'un à côté de l'autre, l'échantillon 2 étant l'échantillon le plus éloigné du centre de rotation de la centrifugeuse. De plus, les échantillons doivent être disposés de façon à optimiser le contact capillaire entre eux. Ensuite, on désature partiellement ces deux échantillons en les soumettant à une centrifugation selon une vitesse de rotation fixée et un temps de rotation suffisant pour permettre une désaturation de l'échantillon 1 (et de l'échantillon par conséquent).

2- <u>Mesure de l'indice de résistivité *RI* de l'échantillon</u>

<u>*Dispositif*</u>

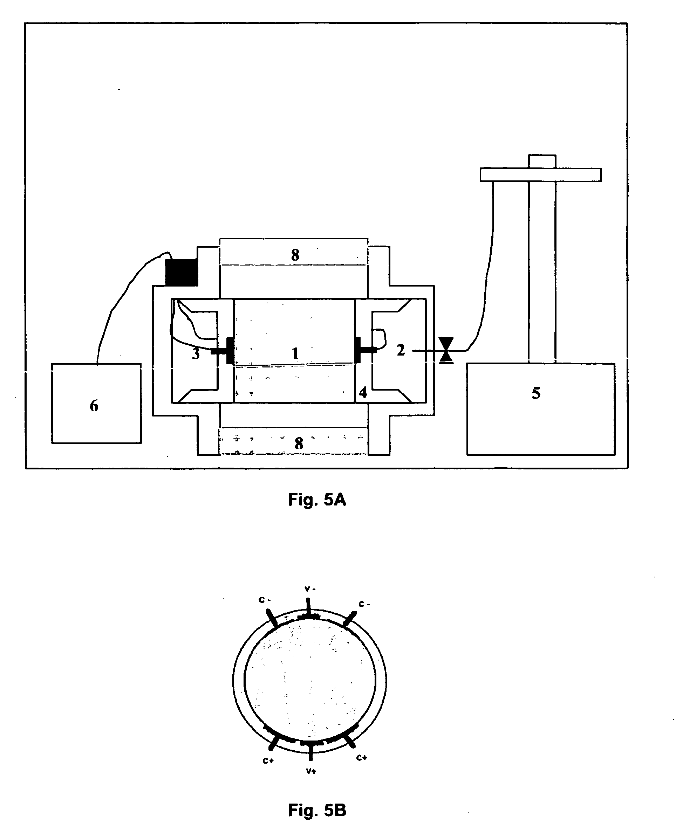

La mesure de résistance est réalisée dans une cellule de mesure de résistivité à électrodes radiale. Un tel dispositif est décrit dans les brevets EP 0 701 128 et FR 2 781 573. Il est composé d'électrodes positionnées radialement autour de l'échantillon. On peut par exemple utiliser un dispositif comportant six électrodes, tel que celui décrit en figure 5A. La figure 5B est une projection verticale de la cellule et des électrodes. La procédure est décrite en référence à ces figures.

Deux couples d'électrodes C+ et C- injectent un courant dans l'échantillon (1) et un autre couple d'électrode V+ et V- mesure le potentiel électrique. Elles sont connectées aux entrées de potentiel et de courant d'un impédance mètre (6) via des fils électriques (3).

Pour éviter le séchage de l'échantillon pendant la mesure, des mousses (8) saturées de saumure sont disposées en haut et en bas pour maintenir une humidité élevée de l'air au contact de l'échantillon.

Une des difficultés pour utiliser ce type d'appareil, est la nécessité d'obtenir un bon contact électrique avec l'échantillon. De plus, il faut que ce contact soit reproductible de façon à ce que les mesures soient elles-mêmes reproductibles.

Les électrodes sont moulées ou insérées dans une gaine en élastomère (4) permettant d'appliquer une pression de confinement. Il peut s'agir d'une gaine en Viton par exemple. Le fluide de confinement contenu dans le compartiment (2) est relié à une pompe (5) remplie d'une huile visqueuse servant à appliquer une pression de confinement fixée à 30 bar. Cette pression nous permet d'avoir un bon contact électrique entre les électrodes et l'échantillon, par suite les champs électriques dans l'échantillon restent quasi constants entre les différentes mesures sur un même échantillon. Ceci nous garantit une condition de mesure constante pour l'indice de résistivité.

Un second problème couramment rencontré, est la perte de grain lors de la manipulation de l'échantillon, conduisant à des erreurs de mesures.

Selon l'invention, pour retirer l'échantillon sans l'endommager, après la mesure électrique, on utilise avantageusement la gaine (4) et la pompe (5). En effet, la pompe peut retirer l'huile dans la cellule, donc produire un écartement des électrodes permettant de retirer sans glissement l'échantillon.

*Mesure*

On injecte un courant entre C+ et C-, on mesure un potentiel entre V+ et V-, et on déduit une résistance R=U/I.

On mesure la partie réelle et la partie imaginaire de la résistance à plusieurs fréquences. La mesure est faite avec un impédance mètre qui détermine l'impédance complexe. On s'affranchit ainsi des effets capacitifs (partie imaginaire). On prend en général la partie réelle à 1kHz pour calculer l'indice de résistivité. La partie imaginaire sert à évaluer la fiabilité de la mesure. À 1 kHz, elle doit être environ 10 fois plus faible que la partie réelle. La mesure se stabilise pendant une dizaine de minutes après l'application de la pression de confinement.

3- Mesure de la saturation

On retire l'échantillon du dispositif. Puis, on mesure la saturation à l'aide d'un appareil RMN qui mesure l'aimantation nucléaire. Un tel appareil est bien connu des spécialistes.

Un noyau atomique peut absorber les rayonnements électromagnétiques d'une fréquence spécifique en présence d'un champ magnétique. Ce phénomène est appelé la Résonance Magnétique Nucléaire (RMN). Dans la description de l'invention, on considère que le noyau d'hydrogène est présent dans la saumure.

Un proton peut passer à un niveau d'énergie plus haut s'il absorbe une quantité d'énergie à la fréquence de Larmor $\omega$, cette dernière étant reliée au champ magnétique $B_0$ dans lequel est placé l'échantillon:

$$\Delta E = \hbar\omega \qquad \text{et} \qquad \omega = \gamma B_0 \qquad (3)$$

où $\hbar$ Est la constante Planck divisée par $2\pi$. $\gamma$ est le rapport gyromagnétique du noyau considéré. $\boldsymbol{B_0}$ est un champ magnétique statique le long l'axe Z. Un autre champ magnétique oscillant $\boldsymbol{B_1}$ est appliqué dans le plan XY. Quand la fréquence de $\boldsymbol{B1}$ approche $\omega$, les protons irradiés passent au niveau d'énergie le plus haut.

Le retour des protons vers le niveau d'énergie inférieur après la suppression du champ $\boldsymbol{B_1}$ s'appelle la relaxation. Les temps caractéristiques nécessaires à un noyau pour revenir à l'équilibre sont le temps de relaxation spin-réseaux noté $T_1$, et le temps de relaxation spin-spin $T_2$. Nous mesurons $T_2$ en général.

L'appareil RMN mesure l'aimantation nucléaire globale M(t) retournant à l'équilibre en fonction du temps à l'aide de la séquence CPMG. La relaxation spin-spin T2 est décomposée en une somme d'exponentielles telle que :

$$M\left(t = 2n_j\tau\right) = \sum_i A_i \exp\left(-\frac{2n_j\tau}{T_{2i}}\right) \quad n_j = 1...n \qquad (4)$$

Les principaux paramètres d'acquisition sont : $\tau$, demi-temps inter écho, par exemple 60 microsecondes, et n, nombres d'échos ou de points de mesure (il varie en fonction des échantillons).

Dans la mesure du possible, on tente d'atteindre un rapport signal sur bruit supérieur à 100.

En général la mesure de l'aimantation totale permet d'obtenir directement le volume poreux Vp en comparant le signal avec une quantité de saumure connue $V_{\text{étalon}}$,

$$V_p = \frac{Mi(Sw = 100\%))}{Mi(saumure\ etalon)} \times V_{étalon} \qquad (5)$$

où Mi est l'aimantation M(t=0).

Dans le système saumure-air, l'air ne produit pas de signal RMN, et on obtient directement les saturations Sw au cours de la centrifugation ou de l'imbibition en faisant le rapport des aimantations Mi selon:

$$S_w = \frac{Mi(Sw))}{Mi(Sw = 100\%))} \qquad (6)$$

La méthode selon l'invention consiste donc à mesurer, à l'aide d'un appareil RMN, les aimantations nucléaires, puis à utiliser la formule (6) afin de déterminer la valeur de la saturation Sw. Il faut donc connaître Mi(Sw =100%)). Les échantillons étant saturés au départ de la méthode, il est nécessaire de réaliser une mesure de l'aimantation nucléaire avant l'étape de désaturation.

**Exemples de mesure**

*1) Grès de Fontainebleau:*

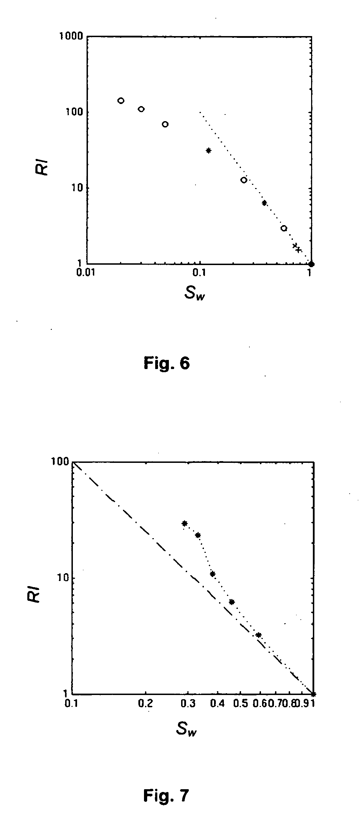

La figure 6 montre le résultat de mesures de résistivité et de saturations en saumure sur deux Grès de Fontainebleau de grande perméabilité (1000 mD). On observe une bonne cohérence entre les deux échantillons. La pente de la courbe $RI = S_w^{-n}$ est proche de 2 au dessus d'une saturation d'environ 0.20. La méthode proposée a permis de montrer qu'en dessous de 0.20, on observe un changement de pente, qui est interprété comme le début d'un régime de conduction dominée par les films.

*2) Calcaire à structure bimodale, cas 1*

Sur une structure complexe tel qu'un calcaire contenant deux tailles de pores, la courbe d'indice de résistivité (figure 7) montre un comportement particulier: on observe une déviation vers le haut. Les profils de saturation montrent que la saturation est uniforme aux différentes étapes de saturation. Les distributions de temps de relaxation montrent deux modes correspondants chacun à une distribution de taille de pore. Lorsque le système poreux est partiellement désaturé, on voit que les grands pores sont vidés en premier, alors que les petits pores restent complètement saturés jusqu'à à une saturation moyenne de 0.38.

*3) Calcaire à structure bimodale, cas 2*

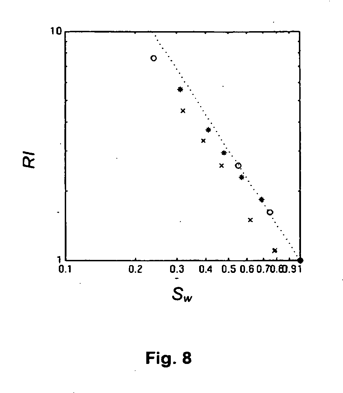

Dans le cas de ce calcaire, la courbe (figure 8) d'indice de résistivité ne présente pas d'anomalies marquantes. Il existe une hystérésis faible en imbibition. Les temps de relaxation montrent que les deux populations sont vidées en même temps dans ce cas. En imbibition, les gros pores restent vides.

**Exemples particuliers**

Dans un exemple (non revendiqué), on complète les mesures effectuées sur les échantillons, par une mesure de distribution de temps de relaxation, à l'aide d'un appareil RMN. Ces mesures de distribution servent à déterminer les pores vidés pour une pression donnée. Cela influence la réponse électrique. L'utilisation des techniques RMN complémente ainsi l'information obtenue et est utile pour l'interprétation de la réponse électrique. En effet, dans des structures complexes comprenant plusieurs populations de tailles de pore (par microporosité et macroporosité), la manière dont les différentes populations se drainent, influence la réponse électrique.

La distribution de temps de relaxation donne une information sur la distribution de taille de pore suivant la relation :

$$\frac{1}{T_2} = \rho_2 \frac{S_p}{V_p} + \frac{1}{T_{2B}} \qquad (7)$$

où $\rho_2$ est le coefficient de relaxivité de surface, et $T_{2B}$ le temps de relaxation propre de la saumure. Le temps de relaxation $T_2$ d'un pore est lié à son rapport surface sur volume (Sp/Vp). La distribution de $T_2$, calculée selon l'équation 4, traduit la distribution du rapport V/S dans le réseau poreux, appelé distribution de "tailles de pores". Ceci est vrai dans le cas d'un échange lent entre les pores. Ainsi, les temps de relaxation les plus longs correspondent aux pores de plus grandes dimensions.

Dans le système saumure-air, le temps de relaxation $T_2$ d'un échantillon partiellement saturé est lié à la surface mouillée par l'eau $S_m$ et à la saturation $S_{wp}$ de ce pore :

$$\frac{1}{T_2} = \rho_2 \frac{S_m}{V_p * S_{wp}} + \frac{1}{T_{2B}} \qquad (8)$$

Selon un autre exemple (non revendiqué), on complète les mesures effectuées sur les carottes, par une mesure de profil de saturation. Ces mesures de profils de saturation permettent de savoir si la saturation est uniforme ou pas : si les échantillons 1 et 2 ne sont pas en contact capillaires, il y aura un profil de saturation sur l'échantillon 1, semblable à 2. Il faut alors recommencer les mesures en améliorant le contact capillaire entre les échantillons.

En utilisant les résultats d'une injection de mercure bien connue des spécialistes, on peut prévoir le profil de saturation en fonction de la vitesse de rotation. En supposant que l'échantillon est mouillable à l'eau, la relation de conversion est la suivante :

$$P_{saumure-air} = P_{Hg} \frac{\gamma_{saumure-air} \cos\theta_{saumure-air}}{\gamma_{Hg} \cos\theta_{Hg}}$$

où $\gamma$ est la tension superficielle et $\theta$ l'angle de contact, les indices désignant le système mercure - air (Hg) et le système air - saumure. A une position et vitesse de rotation donnée par l'équation (1), on calcule la pression obtenue et on cherche la saturation correspondante dans la courbe de pression capillaire air - saumure. Cela nous permet de trouver la saturation locale à chaque position R, et d'obtenir ainsi les profils de saturation (figure 4). On voit que dans l'échantillon 1, la saturation est quasi-uniforme et que le pied capillaire est contenu dans l'échantillon 2.

Profil de saturation

La mesure du profil de saturation est réalisée avec une séquence standard d'imagerie RMN, (une séquence d'écho de Hahn avec gradient pulsé). Lorsqu'un gradient de champ est appliqué, le champ magnétique varie linéairement le long la direction du gradient. La fréquence de Larmor des protons varie proportionnellement le long du gradient magnétique.

$$\omega(y) = \gamma B_0 (1 + Gy) \qquad (9)$$

La fréquence locale code la position des protons suivant la direction du gradient. En reliant la densité de protons mesurée et les positions, on obtient le profil de densité protonique $d_p(z)$ le long la direction du gradient.

Dans le système saumure-air, la mesure du profil de densité $d_p(z)$ permet d'obtenir le profil de saturation Sw(z):

$$S_w(z) = \frac{dp(z)}{dp(z, Sw = 100\%)} \qquad (10)$$

Selon un autre exemple (non revendiqué), on remplace le drainage par centrifugation par une imbibition spontanée. L'échantillon est alors posé dans un bécher dans lequel on ajoute une quantité contrôlée de saumure. La saumure se répand alors dans tout l'échantillon. En répétant cette opération, on peut obtenir les différents paliers de saturation en imbibition. La face d'entrée de la saumure en imbibition correspond à la face de sortie de la saumure en drainage. L'échantillon et la saumure sont à la pression atmosphérique. Les processus de mesure électrique et de mesure RMN sont les mêmes qu'en drainage.

**Applications de la technique de mesure selon l'invention**

Selon le mode de réalisation de l'invention, on utilise la méthode de mesure rapide de la saturation et de l'indice de résistivité d'un milieu poreux, pour définir une relation permettant de déduire la saturation en fonction de la résistivité.
Pour ce faire on réalise plusieurs mesures à partir du même échantillon issu du milieu. A la fin de la première série de mesures, l'échantillon est remis dans la centrifugeuse pour obtenir un autre palier de saturation. On modifie alors la vitesse de rotation $\omega$. Puis on réitère les étapes de mesures, de façon à obtenir différentes saturations, et pour chacune de ces saturations, on mesure l'indice de résistivité et la saturation.
On obtient ainsi plusieurs couples de valeurs de résistivité ($RI$) et de saturation ($S_w$) pour l'échantillon étudié.
A partir de ces couples, on estime une relation permettant de déduire la saturation en fonction de la résistivité. On peut par exemple utiliser la loi d'Archie, et déterminer, grâce aux couples, la valeur de l'indice n :

$$RI = S_w^{-n}$$

avec :

  $RI$ : indice de résistivité

  $S_w$ : saturation en saumure du milieu

  $n$ : un nombre entier

Selon un autre mode de réalisation, la méthode peut être appliquée à la détermination de la saturation en saumure (fluide conducteur) d'une formation souterraine, telle qu'un réservoir pétrolier, ou un réservoir de stockage de gaz, par l'interprétation de mesures électriques, telles que des diagraphies. En effet, cette nouvelle méthode de mesure permet de caractériser très rapidement un grand nombre d'échantillons à un coût faible, en raison de l'utilisation d'une centrifugeuse et d'un montage électrique simple. Bien qu'elle ne permette pas d'explorer des domaines de pression et température proches du réservoir, il est utile d'établir une courbe de référence dans des conditions simplifiées, pour évaluer, par la suite avec des techniques plus contraignantes, les effets de pression et de température. Ainsi, la méthode peut être utilisée dans le but de choisir les échantillons, avant une interprétation plus précise en conditions de pression et de température de la formation. La méthode comporte alors les étapes suivantes :
On extrait des échantillons de la formation. Puis on les sature en saumure. Puis, en utilisant la méthode précédente, on détermine une relation permettant de déduire la saturation en fonction de la résistivité pour chacun des échantillons. On sélectionne alors des échantillons de façon à ce que chacun d'entre eux ait une relation différente des autres échantillons. Par exemple, si l'on choisit d'utiliser la loi d'Archie, on estime une valeur de l'indice « n » pour chaque échantillon. Tous les échantillons ayant une relation commune, c'est-à-dire avec une même valeur de « n », sont regroupés. Puis, on en choisit un seul parmi cette famille d'échantillon. Ces échantillons sélectionnés sont représentatifs d'une corrélation particulière entre la saturation et la résistivité. Ensuite, on mesure l'indice de résistivité et la saturation en saumure de chacun de ces échantillons sélectionnés, à l'aide d'une méthode adaptée à fonctionner selon des conditions de température et de pression propres à la formation souterraine. On peut par exemple utiliser la méthode décrite dans Fleury M., "Advances in Resistivity Measurements using the FRIM Method at Reservoir Conditions. Applications to Carbonates". Proceedings of the International Symposium of the Society of Core Analysts, Septembre 2003, Pau, FRANCE. Enfin, on déduit la saturation de la formation souterraine, en appliquant la relation à au moins une

**EP 2 034 308 B1**

diagraphie de résistivité eléctrique, mesurée à travers la formation. On applique les différentes relations en fonction de la profondeur de la diagraphie et de celle de l'échantillon correspondant, ou à travers un regroupement des différentes mesures appelé "Rock-Typing" non nécessairement reliées de manière simple à la profondeur.

**Revendications**

1. Méthode pour construire une relation permettant de déduire une saturation en un fluide conducteur en fonction d'une résistivité, pour un milieu poreux initialement saturé par ledit fluide, **caractérisée en ce que** :

   a)- on extrait au moins un premier échantillon solide dudit milieu ;
   b)- on place ledit premier échantillon dans une cellule de centrifugation, à côté d'un second échantillon adapté à limiter un pied capillaire dans ledit premier échantillon, les échantillons étant disposés l'un à côté de l'autre de façon à optimiser un contact capillaire entre eux ;
   c)- on désature partiellement les deux échantillons en les soumettant à une centrifugation selon une vitesse de rotation fixée et un temps de rotation suffisant pour permettre une désaturation dudit premier échantillon ;
   d)- on mesure la résistivité du premier échantillon, en le plaçant dans une cellule de mesure de résistivité, adaptée à injecter un courant par l'intermédiaire d'au moins un premier couple d'électrodes, et à mesurer un potentiel entre au moins un second couple d'électrodes, lesdites électrodes étant disposées radialement autours dudit premier échantillon ;
   e)- on détermine la saturation du premier échantillon en mesurant une aimantation nucléaire de l'échantillon à l'aide d'un appareil RMN ; et
   f)- on réitère les étapes b) à e), en modifiant la vitesse de rotation de façon à obtenir plusieurs couples de valeurs de résistivité et de saturation pour ledit premier échantillon, puis on estime une relation permettant de déduire la saturation en fonction de la résistivité.

2. Méthode selon la revendication 1, dans laquelle ledit second échantillon est extrait dudit milieu poreux.

3. Méthode selon l'une des revendications précédentes, dans laquelle on optimise le contact capillaire en intercalant entre les deux échantillons une membrane mouillable audit fluide.

4. Méthode pour mesurer la saturation en un fluide conducteur d'une formation souterraine, dans laquelle :

   - on extrait des échantillons de ladite formation que l'on sature avec ledit fluide ;
   - on détermine une relation permettant de déduire la saturation en fonction de la résistivité pour chacun desdits échantillons, à l'aide de la méthode selon la revendication 1 ;
   - on sélectionne des échantillons de façon à ce que chacun d'entre eux ait une relation différente des autres échantillons ;
   - on mesure l'indice de résistivité et la saturation en fluide de chacun desdits échantillons sélectionnés, à l'aide d'une méthode adaptée à fonctionner selon des conditions de température et de pression propres à ladite formation souterraine ;
   - on réalise au moins une diagraphie de résistivité eléctrique à travers ladite formation ;
   - on en déduit la saturation en fluide dans la formation en appliquant ladite relation.

**Claims**

1. A method for measuring a conducting fluid saturation and a resistivity of a porous medium, initially saturated with said fluid, **characterized in that** it comprises the following stages:

   a) extracting at least a first solid sample from said medium,
   b) placing said first sample into a centrifugation cell, beside a second sample suited to limit a capillary foot in said first sample, the samples being arranged next to one another so as to optimize capillary contact between them,
   c) partly desaturating the two samples by subjecting them to centrifugation with a predetermined rotating speed and a sufficient rotation time to allow desaturation of said first sample,
   d) measuring the resistivity of the first sample by placing it in a resistivity measurement cell suited to inject a current by means of at least a first pair of electrodes and to measure a potential between at least a second pair

9

of electrodes, said electrodes being radially arranged around said first sample,

e) determining the saturation of the first sample by measuring a nuclear magnetization of the sample using an NMR device, and

f) repeating stages b) to e) while modifying the rotating speed so as to obtain several pairs of resistivity and saturation values for said first sample, then estimating a relation allowing to deduce the saturation as a function of the resistivity.

2. A method as claimed in claim 1, wherein said second sample is extracted from said porous medium.

3. A method as claimed in any one of the previous claims, wherein capillary contact is optimized by inserting a membrane wettable by said fluid between the two samples.

4. A method for measuring the conducting fluid saturation of an underground formation, comprising:

- extracting samples from said formation and saturating them with said fluid,
- determining a relation allowing to deduce the saturation as a function of the resistivity for each one of said samples, by means of the method as claimed in claim 1,
- selecting samples in such a way that each one has a different relation from the other samples,
- measuring the resistivity index and the fluid saturation of each one of said samples selected, by means of a method suited to work according to temperature and pressure conditions specific to said underground formation,
- performing at least one electrical resistivity logging operation through said formation,
- deducing therefrom the fluid saturation in the formation by applying said relation.

**Patentansprüche**

1. Verfahren zur Bestimmung einer Beziehung, die es erlaubt, eine Sättigung mit einem leitfähigen Fluid in Abhängigkeit von einem spezifischen Widerstand für ein poröses Medium abzuleiten, das anfangs mit diesem Fluid gesättigt ist, **gekennzeichnet durch**:

a) das Entnehmen mindestens einer ersten festen Probe aus diesem Medium;

b) das Anordnen dieser erste Probe in einer Zentrifugierzelle neben einer zweiten Probe, die geeignet ist, einen Kapillaransatz in dieser ersten Probe zu begrenzen, wobei die Proben derart nebeneinander angeordnet sind, dass ein Kapillarkontakt zwischen beiden optimiert wird;

c) das partielle Entsättigen der beiden Proben, indem sie einer Zentrifugierung mit einer bestimmten Rotationsgeschwindigkeit und Rotationszeit ausgesetzt werden, die ausreicht, um eine Entsättigung der ersten Probe zu erlauben;

d) das Messen eines spezifischen Widerstands der ersten Probe, indem sie in eine Widerstandsmesszelle gelegt wird, die geeignet ist, **durch** mindestens ein erstes Elektrodenpaar einen Strom einzuspeisen und zwischen mindestens einem zweiten Elektrodenpaar ein Potenzial zu messen, wobei diese Elektroden radial um die erste Probe herum angeordnet sind;

e) das Bestimmen der Sättigung der ersten Probe, indem eine Kemmagnetisierung der Probe mithilfe einer MKR-Vorrichtung gemessen wird; und

f) das Wiederholen der Schritte b) bis e), wobei die Rotationsgeschwindigkeit so modifiziert wird, dass für die erste Probe mehrere Paare von Widerstands- und Sättigungswerten erhalten werden, dann das Schätzen einer Beziehung, die es erlaubt, die Sättigung in Abhängigkeit vom spezifischen Widerstand abzuleiten.

2. Verfahren nach Anspruch 1, wobei die zweite Probe aus dem porösen Medium entnommen wird.

3. Verfahren nach einem der vorherigen Ansprüche, wobei der Kapillarkontakt optimiert wird, indem zwischen den beiden Proben eine Membran angeordnet wird, die mit dem leitfähigen Fluid benetzbar ist.

4. Verfahren zur Messung der Sättigung einer unterirdischen Formation mit einem leitfähigen Fluid, umfassend:

- das Entnehmen von Proben aus dieser Formation, die mit diesem Fluid gesättigt werden;
- das Bestimmen, mithilfe des Verfahrens gemäß Anspruch 1, einer Beziehung, die es erlaubt, für jede dieser Proben die Sättigung in Abhängigkeit vom spezifischen Widerstand abzuleiten;
- das Wählen der Proben derart, dass jede davon eine andere Beziehung als die anderen Proben hat;

- das Messen des Widerstandsindexes und der Fluidsättigung jeder der gewählten Proben mithilfe eines Verfahrens, das geeignet ist, unter den in dieser unterirdischen Formation vorherrschenden Temperatur- und Druckbedingungen zu funktionieren;
- as Durchführen mindestens einer Bohrlochmessung des elektrischen Widerstands durch diese Formation hindurch;
- das Ableiten der Fluidsättigung in der Formation anhand dieser Beziehung.

**Fig. 1**

**Fig. 2**

**Fig. 3**

$(R\text{-}R_{max}) / (R_{max}\text{-}R_0)$

**Fig. 4**

**Fig. 5A**

**Fig. 5B**

**Fig. 6**

**Fig. 7**

**Fig. 8**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2864244 A
- EP 0701128 A
- FR 2781573

**Littérature non-brevet citée dans la description**

- **FLEURY M.** Advances in Resistivity Measurements using the FRIM Method at Reservoir Conditions. Applications to Carbonates. *Proceedings of the International Symposium of the Society of Core Analysts,* Septembre 2003